# EUROPÄISCHE PATENTSCHRIFT

(11) **EP 4 028 748 B1**
(45) Veröffentlichungstag und Bekanntmachung des Hinweises auf die Patenterteilung: **30.10.2024**
(21) Anmeldenummer: 20776077.8
(22) Anmeldetag: 08.09.2020
(51) Int. Cl.: G01N 21/05, G01N 21/3504, G01N 21/64, G01N 33/497, A61B 5/087, A61B 5/00, A61B 5/08, A61B 5/083, A61B 5/097, A61M 16/00, A61M 16/08, A61M 16/10

(54) **MESSKÜVETTE UND STRAHLUNGSSENSOR, WELCHE NUR IN VORBESTIMMTER RELATIVORIENTIERUNG LÖSBAR VERBINDBAR SIND**
MEASUREMENT CUVETTE AND RADIATION SENSOR, WHICH CAN BE DETACHABLY CONNECTED ONLY IN A PREDEFINED RELATIVE ORIENTATION
CUVETTE DE MESURE ET CAPTEUR DE RAYONNEMENT POUVANT ÊTRE RELIÉS AMOVIBLES UNIQUEMENT DANS UNE ORIENTATION RELATIVE PRÉDÉFINIE

(30) Priorität: 09.09.2019 DE 102019124107
(43) Veröffentlichungstag der Anmeldung: 20.07.2022
(73) Patentinhaber: Hamilton Medical AG, 7402 Bonaduz (CH)
(72) Erfinder: BREITRUCK, Felix, 9470 Buchs (CH); ROFFLER, Jürg, 7000 Chur (CH); SCHREIBER, Marie-Kristin, 7000 Chur (CH); SCHRANZ, Christoph, 7306 Fläsch (CH)
(74) Vertreter: Ruttensperger Lachnit Trossin Gomoll
(86) Internationale Anmeldenummer: PCT/EP2020/075051
(87) Internationale Veröffentlichungsnummer: WO 2021/048111

(56) Entgegenhaltungen:
- DE-T2- 69 734 401
- US-A- 5 793 044
- US-A- 6 095 986
- US-A1- 2007 261 698

## Beschreibung

Die vorliegende Erfindung betrifft eine Messküvette zur Erfassung wenigstens eines Fluidbestandteils eines die Messküvette durchströmenden Fluids unter Beteiligung von elektromagnetischer Strahlung, gemäß dem Oberbegriff von Anspruch 1

Die vorliegende Erfindung betrifft außerdem ein Strahlungsbauteil, ausgebildet zur Kopplung mit einer Messküvette der vorliegenden Anmeldung, Gemäß dem Oberbegriff von Anspruch 7

Die vorliegende Erfindung betrifft ebenso eine Sensorbaugruppe, welche eine Messküvette der vorliegenden Anmeldung und ein Strahlungsbauteil der vorliegenden Anmeldung umfasst.

Eine gattungsgemäße Messküvette sowie ein gattungsgemäßes Strahlungsbauteil und folglich eine gattungsgemäße Sensorbaugruppe sind aus der DE 697 34 401 T2 bekannt. Diese Messküvette ist zur Messung von Blut-Parametern ausgebildet.

Eine weitere Messküvette ist beispielsweise aus der WO 96/007886 A1 bekannt. Diese Messküvette ist dort in den Figuren 12 und 13 gezeigt. Der Kopplungs- und Erfassungsabschnitt der bekannten Messküvette ist dabei von einem U-förmigen Rahmen eingerahmt, wobei sich der Kopplungs- und Erfassungsabschnitt zwischen den parallelen Schenkeln des U-förmigen Rahmens längs der Basis des U-förmigen Rahmens zwischen diesen Schenkeln erstreckt. Die erste und die zweite Anschlussformation stehen in Gestalt von Anschlussstutzen von den parallelen Schenkeln des U-förmigen Rahmens ab, und zwar jeweils auf der vom Kopplungs- und Erfassungsabschnitt weg weisenden Seite.

Der U-förmige Rahmen ist durch ein U-förmig geformtes flächiges Schalenbauteil gebildet, wobei die jeweiligen Schenkel und die Basis des Schalenbauteils im Wesentlichen eben sind. Die parallelen ebenen Schenkel des U-förmigen Rahmens sind an ihrem basisfernen freien Längsende mit unterschiedlicher Gestalt ausgebildet. Das mit der Messküvette lösbar koppelbare kooperierende Strahlungsbauteil weist an seinen beiden Endseiten bezüglich der geradlinigen Aufnahmeachse Vertiefungen auf, welche mit ebenfalls unterschiedlicher Gestalt komplementär zu je einer Gestalt der Schenkel des U-förmigen Rahmens ausgeformt sind, sodass die freien Längsenden der beiden Schenkel nach dem Schlüssel-Schloss-Prinzip jeweils nur in die ihnen zugeordnete Vertiefung an einer Endseite, nicht jedoch in die jeweils andere Vertiefung an der anderen Endseite passen. Somit bilden die freien Längsenden der beiden parallelen ebenen Schenkel des U-förmigen Rahmens eine Orientierungsstruktur der Messküvette. Ebenso bilden die endseitigen Vertiefungen im Gehäuse des Strahlungsbauteils, welche mit den freien Längsenden der Schenkel formschlüssig zusammenwirken, eine Ausrichtungsstruktur des Strahlungsbauteils. Beide Strukturen sorgen dafür, dass die Messküvette nur in genau einer Relativorientierung bzw. Relativausrichtung mit dem Strahlungsbauteil koppelbar ist.

An ihren basisfernen freien Längsenden weisen die Schenkel des U-förmigen Rahmens jeweils eine Rastnase auf, welche durch Schlitzung des Schenkels längs der Vorsprungsrichtung der Rastnase federnd angeordnet ist. Mit diesen Rastnasen kann die Messküvette überwindbar mit dem Strahlungsbauteil im betriebsbereit gekoppelten Zustand verrastet sein, sodass die Messküvette und das Strahlungsbauteil bis zu einem gewissen Grad trennsicher miteinander koppelt sind.

Die Begriffe "Orientierung" und "Ausrichtung" sind dabei synonym zu verstehen. Es werden lediglich unterschiedliche Begriffe gebraucht, um einmal die Zuordnung zur Messküvette und ein andermal die Zuordnung zum Strahlungsbauteil zu verdeutlichen.

Weitere Messküvetten und Strahlungsbauteile, welche in der oben beschriebenen Art und Weise für eine unverwechselbare Relativorientierung der Messküvette relativ zum Strahlungsbauteil sorgen, sind aus der WO 2007/103855 A2 sowie aus der US 2004/013570 A1 bekannt. Zu diesen Druckschriften gilt das oben zur WO 96/007886 A1 Gesagte entsprechend.

Aus der US 6,095,986 A und aus der US 2007/0261698 A1 ist jeweils eine Messküvette bekannt, welche an ihrer Außenseite Rastmittel zur Verrastung mit einem Strahlungsbauteil aufweist. Die bekannten Rastmittel gestatten eine Verrastung des Strahlungsbauteils an der Messküvette, verhindern jedoch nicht eine Anordnung des Strahlungsbauteils an der Messküvette in unterschiedlichen Orientierungen.

Nachteilig an diesen Lösungen ist, dass die freien Längsenden des U-förmigen Rahmens um eine in ihrer flächigen Erstreckung liegende Biegeachse mit geringer Kraft biegbar sind, sodass ohne weitere Maßnahmen die Schenkel aufgrund ihrer verhältnismäßig geringen Bauteilsteifigkeit selbst bei versuchtem falsch orientiertem Koppeln von Messküvette und Strahlungsbauteil, anstelle das Koppeln mit unerwünschter Orientierung zu verhindern, durch Biegeverformung die Kopplung trotz unerwünschter bzw. falscher Orientierung zumindest insoweit zulassen können, dass sich der Kopplungs- und Erfassungsabschnitt vollständig im Aufnahmeraum befindet. Aus dem gleichen Grunde eines freien Auskragens können die individuell geformten freien Längsenden der U-förmigen Schenkel abbrechen, sodass dann die Messküvette bzw. das Strahlungsbauteil keinerlei Rückmeldung über eine gewünschte korrekte bzw. eine unerwünschte falsche Orientierung mehr geben kann.

Schließlich ist von den Bauteilen: Messküvette und Strahlungsbauteil, das Strahlungsbauteil in der Regel jenes Bauteil, welches mehrfach verwendet wird, während die Messküvette ein Einwegbauteil darstellt, welches nach einem Gebrauch entsorgt wird. Eine Messküvette wird daher in der Regel nur mit einem Strahlungsbauteil gekoppelt, ein Strahlungsbauteil dagegen mit mehreren Messküvetten. Da die verhältnismäßig dünnen Ränder der freien Längsenden der Schenkel des U-förmigen Rahmens der Messküvette mit Flanken der endseitigen Vertiefungen im Gehäuse des Strahlungsbauteils wechselwirken, und da weiterhin die Relativbewegungsrichtung von Messküvette und Strahlungsbauteil zum Einführen des Kopplungs- und Erfassungsabschnitts in den Aufnahmeraum, bzw. gleichbedeutend zum Aufschieben des Strahlungsbauteils auf den Kopplungs- und Erfassungsabschnitt, parallel zur Flächenerstreckung der beiden Schenkel verläuft, treten aufgrund der geringen Randfläche der freien Längsenden an diesen bei vorgegebener Fügekraft hohe Flächenpressungen auf. Wegen dieser hohen Flächenpressungen kann zumindest die Vertiefung im Gehäuse des Strahlungsbauteils durch mehrfach falsch orientiertes Koppeln und Strahlungsbauteil und Messküvette allmählich verformt werden. Dann würde die Ausrichtungsstruktur des Strahlungsbauteils seine Ausrichtungswirkung verlieren.

Es ist daher Aufgabe der vorliegenden Erfindung, eine technische Lehre anzugeben, ein korrekt orientiertes Koppeln von Messküvette und Strahlungsbauteil mit höherer Sicherheit als im Stand der Technik für eine Vielzahl von Kopplungsvorgängen zu gewährleisten.

Diese Aufgabe löst die vorliegende Erfindung gemäß einem ersten Gesichtspunkt durch eine Messküvette mit allen Merkmalen des Anspruchs 1. Bei einer solchen Messküvette ist nicht nur die körperliche Orientierungsstruktur zwischen dem ersten und dem zweiten Längsende des Kopplungs- und Erfassungsabschnitts mit einem sich längs der Längsachse ändernden Abstand zu der Längsachse oder/und mit einer sich längs der Längsachse ändernden, zur Längsachse orthogonalen Abmessung angeordnet und ausgebildet.

Durch die erfindungsgemäße Anordnung oder/und Ausbildung der Orientierungsstruktur verläuft die Orientierungsstruktur mit ihrer größten Verlaufskomponente längs der Längsachse und damit bevorzugt orthogonal zur Kopplungsachse. Das Risiko der Orientierungsstruktur, durch die bei der Herstellung eines betriebsbereiten Zustands längs der Kopplungsachse ausgeübte Fügekraft verformt oder abgebrochen zu werden, ist somit beseitigt oder wenigstens erheblich verringert. Auch die oben im Zusammenhang mit dem Stand der Technik genannten hohen Flächenpressungen können bei dieser Ausgestaltung vermieden werden.

Hohe Flächenpressungen und damit verbundene Verformungen oder sogar Beschädigungen lassen sich dadurch noch sicherer vermeiden, dass sich die Orientierungsstruktur längs der Längsachse über mehr als ein Drittel der Abmessung des Kopplungs- und Erfassungsabschnitts erstreckt. Je größer die Abmessung der Orientierungsstruktur längs der Längsachse ist, desto größer ist der Bauteilabschnitt der Messküvette, über welchen sich eine zur Kopplung der Messküvette mit dem Strahlungsbauteil ausgeübte Fügekraft verteilen kann. Daher erstreckt sich die Orientierungsstruktur bevorzugt über mehr als zwei Drittel der Abmessung, besonders bevorzugt über die gesamte Abmessung des Kopplungs- und Erfassungsabschnitts, oder sogar noch darüber hinaus. Die Orientierungsstruktur kann sich zur starken Verringerung der beim Koppeln auf sie wirkende mechanische Belastung über beide Längsenden des Kopplungs- und Erfassungsabschnitt hinaus erstrecken.

Die Längsachse fällt dann, wenn die Kanalbahn als geradlinige Kanalachse ausgebildet ist, mit der Kanalachse zusammen. Ist die Kanalbahn innerhalb des Kopplungs- und Erfassungsabschnitts gekrümmt, dann kann die Kanalachse eine Tangente an die gekrümmte Kanalbahn sein. Allerdings zeigt die WO 96/007886 A1, dass die Kanalbahn im Kopplungs- und Erfassungsabschnitt mehrfach umgelenkt sein kann. Dann, wenn die Kanalbahn im Kopplungs- und Erfassungsabschnitt umgelenkt ist, jedoch die Kanalbahnrichtungen an beiden Längsenden des Kanalbereichs des Kopplungs-und Erfassungsabschnitts parallel oder kollinear sind, wie dies etwa in der WO 96/007886 A1 der Fall ist, ist die Längsachse in der Regel parallel bzw. kollinear zu den Kanalbahnrichtungen an den Längsenden des Kanalbereichs.

Grundsätzlich ist der Kopplungs- und Erfassungsabschnitt durch Relativbewegung längs einer Kopplungsachse mit dem Strahlungsbauteil koppelbar. Das Strahlungsbauteil umgibt den Kopplungs- und Erfassungsabschnitt in der Regel an drei aufeinanderfolgenden Seiten, sodass anstelle einer vierten Seite eine Einführöffnung am Strahlungsbauteil verbleibt, durch welche hindurch die Messküvette bzw. ihr Kopplungs- und Erfassungsabschnitt längs der Kopplungsachse in den Aufnahmeraum des Strahlungsbauteils einführbar ist. Beachtet man anstelle der Messküvette das Strahlungsbauteil als bewegtes Bauteil, ist das Strahlungsbauteil längs der Kopplungsachse auf den Kopplungs- und Erfassungsabschnitt aufschiebbar, wobei die Einführöffnung bei dieser Aufschiebebewegung vorauseilt. Die Kopplungsachse, längs welcher eine relative Kopplungsbewegung von Messküvette und Strahlungsbauteil zur Herstellung eines betriebsbereiten Zustandes der die Messküvette und das Strahlungsbauteil umfassenden Sensorbaugruppe verläuft, ist sowohl der Messküvette für sich alleine genommen als auch dem Strahlungsbauteil für sich alleine genommen ohne weiteres anzusehen.

Die Längsachse ist dann bevorzugt orthogonal zur Kopplungsachse und verläuft längs der globalen Durchströmungsrichtung, längs welcher die Messküvette zwischen ihrem ersten und ihrem zweiten Längsende von Fluid durchströmbar ist. Eine sowohl zur Längsachse als auch zur Kopplungsachse orthogonale dritte Achse vervollständigt die Längsachse und die Kopplungsachse zu einem kartesischen Achsensystem. Diese dritte Achse ist in der Regel die Einstrahlachse, längs welcher elektromagnetische Strahlung vom Strahlungsbauteil in den Kanalbereich im Kopplungs-und Erfassungsabschnitt eingestrahlt wird.

Im Zweifel soll die Längsachse den Kanalbereich zentral durchsetzend verstanden werden, also etwa als Spur von Flächenmittelpunkten. Auf die genaue Lage der Längsachse kommt es jedoch nicht an. Es genügt, die Orientierung der Längsachse festzustellen.

Die Orientierungsstruktur kann in der gewünschten Orientierung relativ zum Strahlungsbauteil kollisionsfrei dem Strahlungsbauteil angenähert werden, während in einer unerwünschten bzw. falschen Orientierung die Orientierungsstruktur mit einem Bauteilabschnitt des Strahlungsbauteils kollidiert, bevor eine betriebsbereite Kopplung von Messküvette und Strahlungsbauteil erreicht ist. Die körperliche Kollision hindert eine weitere Relativbewegung von Messküvette und Strahlungsbauteil in einen betriebsbereiten Kopplungszustand.

Im Stand der Technik verläuft der Kopplungs- und Erfassungsabschnitt mit all seinen mit dem Strahlungsbauteil körperlich wechselwirkenden Bauteilabschnitten parallel zur Längsachse, sodass der Kopplungs- und Erfassungsabschnitt für sich alleine genommen in den Vorrichtungen des Standes der Technik, mit Ausnahme der aus der DE 697 34 401 T2 bekannten Messküvette und Strahlungsbauteil in mehr als einer Orientierung, wenigstens in zwei bezüglich der Kopplungsachse um 180° verdrehten Orientierungen mit dem Strahlungsbauteil koppelbar ist. Durch die oben beschriebene Orientierungsstruktur wird diese Symmetrie des Kopplungs- und Erfassungsabschnitts bezüglich der Kopplungsachse gebrochen. Der Kopplungs- und Erfassungsabschnitt der Messküvette der vorliegenden Erfindung ist hinsichtlich seiner Außenabmessungen nicht bezüglich einer Rotation um die Kopplungsachse um 180° invariant.

In der Regel ist der Kopplungs- und Erfassungsabschnitt durch einen Küvettenabschnitt mit drei in Umfangsrichtung um die Längsachse aufeinanderfolgenden und paarweise zueinander abgewinkelt angeordneten Wänden gebildet, wobei die Wände in einer Schnittansicht in einer zur Längsachse orthogonalen Schnittebene eine U-Gestalt aufweisen. Von einer Basiswand der U-Gestalt steht an beiden Endbereichen je eine Seitenwand ab. Bevorzugt liegt das Strahlungsbauteil im betriebsbereiten Zustand des mit der Messküvette gekoppelten Strahlungsbauteils wenigstens an den vom Kanalbereich weg weisenden Außenseiten der von der Basiswand abstehenden und mit Abstand voneinander angeordneten Seitenwänden mit Berührkontakt an.

In einer bevorzugten Ausführungsform sind zumindest die am weitesten vom Kanalbereich weg vorstehenden Außenflächenbereiche der beiden Seitenwände eben sowie zueinander und zur Längsachse parallel, um eine spielfreie Anordnung im Aufnahmeraum des Strahlungsbauteils zu erleichtern. Eine zu den ebenen Außenflächenbereichen der Seitenwände orthogonale Achse als Einstrahlachse ist dann zugleich zur Längsachse orthogonal. Die Kopplungsachse ist ebenfalls zu den Außenseiten der Seitenwände parallel und zur Längsachse - sowie unvermeidlich auch zur Einstrahlachse - orthogonal. Bevorzugt ist die Außenfläche zu einer den Abstand zwischen den beiden Seitenwänden überbrückenden Basiswand ebenfalls eben und zur Kopplungsachse orthogonal.

Die Orientierungsstruktur kann grundsätzlich durch den Kanalbereich einfassende Wände des Kopplungs- und Erfassungsabschnitts gebildet sein. Da jedoch aus Gründen erhöhter Genauigkeit des damit erhaltenen Messsignals zur Längsachse und zueinander parallele Seitenwände mit konstanter Wanddicke bevorzugt sind, um eine Brechung oder Beugung der in den Kanalbereich einstrahlenden bzw. den Kanalbereich durchstrahlenden elektromagnetischen Strahlung zu vermeiden, ist die Orientierungsstruktur bevorzugt mit Abstand von den Kanalbereich einfassenden Wänden angeordnet. Es ist jedoch nicht ausgeschlossen, dass dann, wenn lediglich ein bezüglich der Längsachse zentraler Bereich des Kopplungs- und Erfassungsabschnitts für elektromagnetische Strahlung des Strahlungsbauteils durchlässig ausgebildet ist, oder nur in diesem zentralen Bereich eine Durchlässigkeit messtechnisch ausgenutzt wird, ein bezüglich der Längsachse diesem Durchlässigkeitsbereich benachbarter Bereich von den oben genannten den Kanalbereich einfassenden Seitenwänden des Kopplungs- und Erfassungsabschnitts die Orientierungsstruktur ausbildet oder zu dieser beiträgt. Die Orientierungsstruktur kann dann von der Außenseite der Seitenwände auf einer Seite oder beiderseits des Durchlässigkeitsbereichs gebildet sein. Beispielsweise kann die Außenseite wenigstens einer Seitenwand auf einer Seite oder beiderseits des Durchlässigkeitsbereichs um eine zur Kopplungsachse, gegebenenfalls auch um zur Längsachse, parallele Achse wenigstens abschnittsweise geneigt oder gestuft ausgebildet sein.

Ganz grundsätzlich gilt, dass die Orientierungsstruktur derart ausgebildet sein kann, dass sich ihr Abstand zur Längsachse oder/und die zur Längsachse orthogonale Abmessung kontinuierlich oder/und stufenweise ändert. Der Vorteil einer stufenweisen Änderung liegt in dem hierfür geringen Platzbedarf. Der Vorteil einer kontinuierlichen Änderung liegt in der leichten Erkennbarkeit der gewünschten Orientierung und in der über einen größeren Bereich verteilten Auswirkung der Fügekraft beim Fügen mit einem Strahlungsbauteil.

Um bei einer versehentlichen fehlerhaften Orientierung unerwünschte Biege- oder Torsionsmomente auf die Orientierungsstruktur aufgrund eines Abstands längs der Längsachse zwischen zwei Abschnitten mit unterschiedlichem Abstand zur Längsachse bzw. mit unterschiedlicher zur Längsachse orthogonaler Abmessung zu vermeiden, ist bevorzugt, wenn die Orientierungsstruktur wenigstens zwei Abschnitte mit unterschiedlichem Abstand zur Längsachse oder/und mit unterschiedlicher zur Längsachse orthogonaler Abmessung aufweist, wobei die Abschnitte längs der Längsachse lückenlos aufeinander folgen.

Wie oben bereits ausgeführt wurde kann der Kopplungs- und Erfassungsabschnitt zwei längs der Längsachse verlaufende, orthogonal zur Längsachse mit Abstand voneinander angeordnete Wände aufweisen, welche den Kanalbereich der Messküvette begrenzen. Diese Wände können die oben genannten Seitenwände sein. Bevorzugt sind die Wände zueinander parallel. Aus Gründen einfacher Fertigung und aus Gründen einer möglichst geringen Beeinflussung der wenigstens eine Seitenwand, vorzugsweise beide Seitenwände durchstrahlenden elektromagnetischen Strahlung weisen die beiden Seitenwände bevorzugt eine ebene Außenfläche auf. In der Regel weisen die Seitenwände Durchgangsöffnungen für den Durchtritt elektromagnetischer Strahlung auf. Diese können mit einem von den übrigen Seitenwandabschnitten verschiedenen Material verschlossen sein, das für die jeweils gewünschte elektromagnetische Strahlung durchlässig ist.

Zur Vermeidung einer Störung der unmittelbar den Kanalbereich begrenzenden Wände des Kopplungs- und Erfassungsabschnitts durch die Orientierungsstruktur weist die Orientierungsstruktur bevorzugt einen längs der Längsachse verlaufenden Randabschnitt auf, welcher bezüglich der Längsachse geneigt ist. Der Randabschnitt liegt bevorzugt mit Abstand von Wänden, welche den Kanalbereich begrenzen. Bei dem Abstand handelt sich um einen Abstand in einer Richtung orthogonal zur Längsachse, da die Orientierungsstruktur in jenem Längsachsenbereich angeordnet ist, in welchem sich der Kopplungs- und Erfassungsabschnitt befindet. Der Abstand ist vorzugsweise auch orthogonal zur Kopplungsachse orientiert.

Bevorzugt soll eine Bedienperson bereits vor einem Aufschieben des Strahlungsbauteils auf den Kopplungs- und Erfassungsabschnitt der Messküvette die gewünschte richtige Orientierung der Messküvette relativ zum Strahlungsbauteil erkennen können. Dies geht umso einfacher, je deutlicher die gewünschte Orientierung für eine Bedienperson erkennbar ist. Die Orientierungsstruktur einer vorteilhaft weitergebildeten Messküvette, welche selbst bei schlechter Beleuchtung oder im Dunkeln haptisch richtig orientiert werden kann, weist zwei in einem gemeinsamen Längsbereich längs der Längsachse verlaufende Rand abschnitte auf, und zwar auf jeder Seite des Kanalbereichs je einen, wobei jeder der zwei Randabschnitte bezüglich der Längsachse geneigt ist, wobei vorzugsweise sich der orthogonal zur Längsachse zu messende Abstand der beiden Randabschnitte voneinander längs der Längsachse ändert, besonders bevorzugt kontinuierlich ändert. Beispielsweise können die beiden Randabschnitte längs der Längsachse konvergierend angeordnet sein.

Die Randabschnitte können Randabschnitte eines schalen- oder plattenförmigen Bauteilabschnitts des Kopplungs- und Erfassungsabschnitts sein. Dieser schalen- oder plattenförmige Bauteilabschnitt kann eben sein oder/und kann eine Krümmung aufweisen. Die Krümmung kann eine Krümmung um eine zur Längsachse parallele Krümmungsachse oder/und kann eine Krümmung um eine zur Einstrahlachse parallele Krümmungsachse sein. Bevorzugt liegt der Kanalbereich des Kopplungs- und Erfassungsabschnitts vollständig auf einer Seite des schalen- oder plattenförmigen Bauteilabschnitts, sodass der Bauteilabschnitt nach Herstellung einer betriebsbereiten Kopplung mit dem Strahlungsbauteil eine Außenfläche des Gehäuses des Strahlungsbauteils ergänzen kann.

Zur Sicherung einer einmal zwischen Messküvette und Strahlungsbauteil hergestellten Kopplung kann die Orientierungsstruktur Rastmittel tragen, welche zur Verrastung mit Verrastungsmitteln am Strahlungsbauteil ausgebildet sind. Die Rastmittel können ein Rastvorsprung oder eine Rastausnehmung sein. Die Verrastungsmittel weisen dann die jeweils andere Gestalt aus Rastvorsprung und Rastausnehmung auf, sodass in der Kombination von Rastmittel und Verrastungsmittel ein Rastvorsprung und eine Rastausnehmung zur Herstellung einer Verrastung formschlüssig zusammenwirken.

Bevorzugt sind die Rastmittel im Bereich des wenigstens einen Randabschnitts vorgesehen, sodass eine Verrastung mit den Verrastungsmittel sicher nur bei korrekter Orientierung herstellbar ist. Damit wird vermieden, dass ein entfernt von der Orientierungsstruktur vorgesehenes Rastmittel bei ausreichend ausgeübter Fügekraft aufgrund einer zur Herstellung einer Verrastung funktionsnotwendig ausgebildeten Relativbeweglichkeit von Rastmittel und Verrastungsmittel selbst dann in eine Verrastung gelangt, obwohl die Orientierungsstruktur in diesem Fall bestimmungsgemäß mit einem Bauteilabschnitt des Strahlungsbauteils kollidiert. Je näher die Verrastungsmittel an der Orientierungsstruktur, vorliegend bevorzugt am Randabschnitt, ausgebildet sind, desto weniger können dazwischenliegende und sich möglicherweise verformende Bauteile und Bauteilabschnitte trotz unzutreffender Orientierung eine im Grunde unerwünschte Verrastung herbeiführen.

Bevorzugt befindet sich der Kanalbereich auf einer Seite des Randabschnitts bzw. auf einer Seite des den Randabschnitt aufweisenden schalen- oder plattenförmigen Bauteilabschnitts. Daher stehen bevorzugt auch die Rastmittel bezüglich des wenigstens einen Randabschnitts von dem wenigstens einen Randabschnitt zu derselben Seite hin ab, auf welcher sich der unter Beteiligung der beiden Wände begrenzte Kanalbereich der Messküvette befindet.

Bevorzugt sind die Rastmittel federnd, und zwar besonders bevorzugt längs einer Vorkragrichtung eines Rastvorsprungs oder längs einer Vertiefungsrichtung einer Rastausnehmung federnd, an der Messküvette vorgesehen, um das Herstellen und das Lösen einer Verrastung für eine Bedienperson zu erleichtern. Besonders bevorzugt ist der ein Rastmittel tragende Randabschnitt selbst als Feder ausgebildet, beispielsweise durch Schlitzung eines den Randabschnitt aufweisenden schalen- oder plattenförmigen Bauteilabschnitts. Die Schlitzung verläuft vorzugsweise parallel zum Randabschnitt, sodass der Randabschnitt eine an beiden Längsenden eingespannte Blattfeder oder Balkenfeder bildet.

Gemäß einem zweiten, mit dem ersten inhaltlich in Wechselwirkungsbeziehung stehenden Gesichtspunkt der vorliegenden Erfindung löst die vorliegende Erfindung die oben genannte Aufgabe auch durch ein Strahlungsbauteil gemäß Anspruch 7.

Das Strahlungsbauteil ist längs der Aufnahmeachse vom Aufnahmeraum durchsetzt, d. h. diese erstreckt sich längs der Aufnahmeachse zwischen zwei Endseiten des Gehäuses des Strahlungsbauteils. Der Aufnahmeraum ist von drei Seiten, in der Regel U-förmig, durch das Strahlungsbauteil umgeben. Eine vierte, offene Seite bildet eine Einführöffnung durch welche hindurch der Kopplungs- und Erfassungsabschnitt der Messküvette in den Aufnahmeraum eingeführt werden kann. Die oben bereits beschriebene Kopplungsachse ist daher bevorzugt orthogonal auch zur Aufnahmeachse. Im betriebsbereiten Zustand ist das Strahlungsbauteil rittlings am Kopplungs- und Erfassungsabschnitt angeordnet, welchen es auf drei Seiten umgibt, wobei das Strahlungsbauteil bevorzugt an zwei längs der Aufnahmeachse verlaufenden Seitenwänden des Kopplungs- und Erfassungsabschnitts berührend anliegt. Eine zur Kopplungsachse und zur Aufnahmeachse orthogonale dritte Achse an dem Strahlungsbauteil ist eine Strahlungsachse längs welcher bevorzugt elektromagnetische Strahlung den Aufnahmeraum durchstrahlen kann. Im betriebsbereit gekoppelten Zustand fallen die Aufnahmeachse und die Längsachse einerseits sowie die Strahlungsachse und die Einstrahlachse andererseits zusammen, sind also zueinander parallel oder kollinear.

Das Strahlungsbauteil umfasst eine Strahlungsquelle, welche bevorzugt seitlich neben dem Aufnahmeraum in einem Kompartiment des Strahlungsbauteils aufgenommen ist. Das Strahlungsbauteil umfasst bevorzugt zusätzlich einen Sensor, welcher für elektromagnetische Strahlung empfindlich ist und ein Ausgabesignal ausgibt, das die auf den Sensor einstrahlende elektromagnetische Strahlung, je nach Art des Sensors, hinsichtlich ihrer Phasenverschiebung bezüglich einer Referenzstrahlung oder/und ihrer Intensität repräsentiert. Der Sensor kann im selben Bereich des Strahlungsbauteils angeordnet sein wie die Strahlungsquelle, etwa dann, wenn der Sensor reflektierte elektromagnetische Strahlung erfassen soll, oder etwa dann, wenn der Sensor eine von der elektromagnetischen Strahlungsquelle verschiedene angeregte elektromagnetische Strahlung erfassen soll, wie es beispielsweise für fluoreszenzspektroskopische Erfassungsverfahren der Fall ist.

Bevorzugt umfasst oder ist die Strahlungsquelle dann, wenn das Strahlungsbauteil auch oder ausschließlich zur Erfassung eines CO₂-Anteils in einem Fluid ausgebildet ist, eine Infrarot-Strahlungsquelle. Das Strahlungsbauteil ist dann bevorzugt ein nichtdispersiver Infrarotsensor. Zusätzlich oder alternativ kann das Strahlungsbauteil ein fluoreszenzspektroskopischer Sensor sein, dessen Strahlungsquelle ein Luminophor im Kopplungs- und Erfassungsabschnitt der Messküvette zum Abstrahlen einer Anregungsstrahlung anregt, welche Anregungsstrahlung durch einen vorbestimmten Gasanteil des die Messküvette durchströmenden Fluids wieder abgelöscht wird. Auf diese Weise kann beispielsweise Sauerstoff in dem Fluid nachgewiesen und quantifiziert werden. Ein bevorzugter Anwendungsfall des Strahlungsbauteils und der Messküvette ist daher deren Einsatz in einer Atemgasleitung einer Beatmungsvorrichtung zur wenigstens teilweisen künstlichen Beatmung von menschlichen oder tierischen Patienten. Bevorzugt wird sie in einem Abschnitt der Atemgasleitung angeordnet, welcher sowohl von inspiratorischem als auch von exspiratorischem Atemgas durchströmt wird, sodass mit dem Strahlungsbauteil wenigstens ein Gasbestandteil sowohl im exspiratorischen als auch im inspiratorischen Atemgas nachweisbar ist. Dies ist vor allem für die Erfassung von CO₂ als Stoffwechselprodukt vorteilhaft, da so durch den Vergleich von inspiratorischem und exspiratorischem CO₂-Gehalt auf die Stoffwechselfähigkeit des beatmeten Patienten geschlossen werden kann.

Da im betriebsbereit gekoppelten Zustand von Messküvette und Strahlungsbauteil die Orientierungsstruktur im Kopplungs- und Erfassungsabschnitt der Messküvette mit der Ausrichtungsstruktur des Strahlungsbauteils körperlich durch Anlageeingriff oder Formschlusseingriff wechselwirkt, vorzugsweise nach dem Schlüssel-Schloss-Prinzip, indem eine Struktur aus Orientierungsstruktur und Ausrichtungsstruktur, vorzugsweise die am Gehäuse des Strahlungsbauteils ausgebildete Ausrichtungsstruktur, wenigstens einen Randabschnitt einer Vertiefung definiert, in welche die jeweils andere Struktur, vorzugsweise die Orientierungsstruktur, nur bei entsprechend vorbestimmter gewünschter Orientierung hineinpasst, gilt das oben zur Orientierungsstruktur Gesagte mutatis mutandis auch für die Ausrichtungsstruktur des Strahlungsbauteils. Dabei ist vor allem die Längsachse des Kopplungs- und Erfassungsabschnitts der Messküvette durch die Aufnahmeachse des Aufnahmeraums des Strahlungsbauteils sowie die Einstrahlachse durch die Strahlungsachse zu ersetzen.

So ist aus Gründen einer vorteilhaften niedrigen mechanischen Belastung im Falle einer falschen Relativorientierung zwischen Messküvette und Strahlungsbauteil erfindungsgemäß vorgesehen, wenn sich die Ausrichtungsstruktur längs der Aufnahmeachse über mehr als ein Drittel der Abmessung des Aufnahmeraums erstreckt. Vorzugsweise erstreckt sich die Ausrichtungsstruktur über mehr als zwei Drittel der Abmessung, besonders bevorzugt über die gesamte Abmessung des Aufnahmeraums. Wie die Orientierungsstruktur kann sich auch die Ausrichtungsstruktur sogar zu einer oder zu beiden Seiten über ein Längsende des Aufnahmeraums hinaus erstrecken.

Bevorzugt erstreckt sich die Orientierungsstruktur hauptsächlich längs der Längsachse des Kopplungs- und Erfassungsabschnitts, d. h. ihre betragsmäßig größte Verlaufskomponente in einem räumlichen kartesischen Koordinatensystem mit Komponentenachsen parallel zur Längsachse, parallel zur Kopplungsachse und parallel zur Einstrahlachse, ist die Komponente parallel zur Längsachse. Für die Ausrichtungsstruktur gilt im Wesentlichen das gleiche, nur dass hier anstelle der Längsachse die Aufnahmeachse und anstelle der Einstrahlachse die Strahlungsachse anzuwenden ist.

Der Abstand der Ausrichtungsstruktur zur Aufnahmeachse oder/und die zur Aufnahmeachse orthogonale Abmessung kann sich, wie oben bereits für die Orientierungsstruktur ausgeführt, kontinuierlich oder/und stufenweise ändern.

Ebenso kann die Ausrichtungsstruktur zur möglichst sicheren Anordenbarkeit der Messküvette am Strahlungsbauteil nur in der vorbestimmten gewünschten Orientierung wenigstens zwei Abschnitte mit unterschiedlichem Abstand zur Aufnahmeachse oder/und mit unterschiedlicher zur Aufnahmeachse orthogonaler Abmessung aufweisen, wobei die Abschnitte längs der Aufnahmeachse lückenlos aufeinander folgen.

Eine flächenmäßig große und daher für eine Bedienperson nicht nur sichtbare sondern auch fühlbare Ausrichtungsstruktur, die eine korrekte Ausrichtung des Strahlungsbauteils auch im Dunkeln oder ohne Hinsehen ermöglicht, kann dadurch realisiert sein, dass an einem Gehäuse des Strahlungsbauteils wenigstens im Bereich einer im Wesentlichen parallel zur Aufnahmeachse verlaufenden, das Strahlungsbauteil längs der Aufnahmeachse vollständig durchsetzenden Einführöffnung eine an die Einführöffnung angrenzende Vertiefung ausgebildet ist, wobei die Ausrichtungsstruktur an oder in dieser Vertiefung ausgebildet ist.

Nun kann grundsätzlich die Vertiefung nach Erwägungen ausgebildet sein, die für die korrekte Ausrichtung des Strahlungsbauteils relativ zur Messküvette nicht notwendigerweise eine Rolle spielen. Es reicht zur Sicherstellung einer Anordenbarkeit der Messküvette relativ zum Strahlungsbauteil in nur einer gewünschten Ausrichtung bzw. Orientierung aus, wenn die Ausrichtungsstruktur eine die Vertiefung begrenzende Flanke umfasst oder ist. Die Höhe der Flanke kann dabei verglichen mit der Gesamtabmessung des Strahlungsbauteils gering ausgebildet sein. Es reicht aus, wenn die Flanke einen halben Millimeter bis 2 mm Höhe aufweist. Die Flanke kann außerdem bezüglich der Kopplungsachse geneigt angeordnet sein, sodass sie als Einführschräge wirkt.

Zur Aufnahme einer möglichst großen und daher leicht und sicher wahrnehmbaren Orientierungsstruktur im Bereich des Kopplungs- und Erfassungsabschnitts der Messküvette in der Vertiefung am Gehäuse des Strahlungsbauteils kann die Ausrichtungsstruktur beiderseits der Einführöffnung je eine die Vertiefung begrenzende Flanke umfassen oder sein, wobei bevorzugt der orthogonal zur Aufnahmeachse zu messende Abstand der Flanken sich längs der Aufnahmeachse ändert, besonders bevorzugt kontinuierlich ändert.

Zur sicheren, vorzugsweise überwindbaren, Festlegung von Messküvette und Strahlungsbauteil aneinander kann das Strahlungsbauteil Verrastungsmittel zur Verrastung der Messküvette im betriebsbereiten Zustand aufweisen. Die Verrastungsmittel des Strahlungsbauteils treten bei betriebsbereiter Kopplung von Messküvette und Strahlungsbauteil vorzugsweise in formschlüssigen Rasteingriff mit den Rastmitteln an der Orientierungsstruktur der Messküvette.

Vorteilhaft können die Verrastungsmittel in der wenigstens einen Flanke angeordnet oder ausgebildet sein, sodass sie nicht über das übrige Gehäuse des Strahlungsbauteils vorstehen und so keine Verletzungen hervorrufen oder Beschädigungen des Strahlungsbauteils provozieren. Auch die Verrastungsmittel können einen Verrastungsvorsprung oder eine Verrastungsausnehmung aufweisen. In der Regel werden die Verrastungsmittel und die Rastmittel komplementäre Formationen zur Herstellung und Lösung eines Rasteingriffs zwischen diesen Mitteln aufweisen.

Schließlich löst die vorliegende Erfindung die oben genannte Aufgabe auch durch eine Sensorbaugruppe, umfassend eine Messküvette, wie sie oben beschrieben und weitergebildet ist, und ein Strahlungsbauteil, wie es oben beschrieben und weitergebildet ist. Bei dieser Sensorbaugruppe sind im betriebsbereiten Zustand, in welchem die Messküvette und das Strahlungsbauteil lösbar miteinander gekoppelt sind, die Längsachse und die Aufnahmeachse in einem gemeinsamen Raumbereich parallel oder kollinear zueinander. Die Orientierungsstruktur und die Ausrichtungsstruktur stehen zumindest abschnittsweise, vorzugsweise vollständig, in Formschluss- oder Anlageeingriff miteinander.

Vorteilhafterweise können ein die Orientierungsstruktur aufweisender küvettenseitiger Bauteilabschnitt und ein die Ausrichtungsstruktur aufweisender strahlungsbauteilseitiger Bauteilabschnitt im betriebsbereit gekoppelten Zustand an der Anlagestelle von Orientierungsstruktur und Ausrichtungsstruktur einen bündigen Außenflächenbereich der Sensorbaugruppe bilden. Dies ist vor allem dann interessant, wenn das Gehäuse des Strahlungsbauteils die oben genannte Vertiefung aufweist, deren Berandung im Längserstreckungsbereich des Aufnahmeraums längs der Aufnahmeachse zumindest abschnittsweise, vorzugsweise vollständig, von der Ausrichtungsstruktur gebildet ist. Dann kann der die Orientierungsstruktur aufweisende küvettenseitige Bauteilabschnitt die Vertiefung im Bereich des Aufnahmeraums gleichsam auffüllen. Im betriebsbereit gekoppelten Zustand von Messküvette und Strahlungsbauteil bilden beide Komponenten zusammen einen Körper mit im Wesentlichen glatter Oberfläche, von welcher an der Vorderseite und an der Rückseite des Strahlungsbauteils bzw. der Sensorbaugruppe lediglich die Anschlussformationen der Messküvette vorstehen.

Die Vertiefung mit der sie wenigstens abschnittsweise berandenden Ausrichtungsstruktur ist vorzugsweise an der Unterseite des Strahlungsbauteils ausgebildet, an welcher auch die oben genannte Einführöffnung ausgebildet ist.

Die vorliegende Erfindung wird nachfolgend anhand der beiliegenden Zeichnungen näher erläutert werden. Es stellt dar:
- Fig. 1: eine grobschematische perspektivische Ansicht einer erfindungsgemäßen Ausführungsform einer Messküvette der vorliegenden Anmeldung,
- Fig. 2A: eine grobschematische Seitenaufrissansicht der Messküvette von Figur 1,
- Fig. 2B: eine grobschematische Draufsicht der Messküvette von Figur 1,
- Fig. 3A: eine grobschematische axiale Ansicht der Messküvette der Figuren 1, 2A und 2B aus der Blickrichtung des Pfeils IIIA von Figur 2A,
- Fig. 3B: eine grobschematische axiale Ansicht der Messküvette der Figuren 1, 2A und 2B aus der Blickrichtung des Pfeils IIIB von Figur 2B,
- Fig. 4A: eine grobschematische Schnittansicht der Messküvette der Figuren 1 bis 3B längs der Schnittebene IVA-IVA von Figur 2A,
- Fig. 4B: eine grobschematische Schnittansicht der Messküvette der Figuren 1 bis 4A längs der Schnittebene IVB-IVB von Figur 2B,
- Fig. 5: eine grobschematische perspektivische Ansicht einer erfindungsgemäßen Ausführungsform eines Strahlungsbauteils der vorliegenden Anmeldung,
- Fig. 6: eine grobschematische perspektivische Ansicht einer erfindungsgemäßen Sensorbaugruppe der vorliegenden Anmeldung, umfassend das Strahlungsbauteil von Figur 5 und die Messküvette der Figuren 1 bis 4B während eines Kopplungsvorgangs zur Kopplung der Messküvette mit dem Strahlungsbauteil,
- Fig. 7: eine weitere grobschematische perspektivische Ansicht der Sensorbaugruppe von Figur 6 während des Kopplungsvorgangs aus anderer Blickrichtung, und
- Fig. 8: eine grobschematische perspektivische Ansicht der Sensorbaugruppe der Figuren 6 und 7 aus der Betrachterposition der Figuren 5 und 7 nach dem Ende des Kopplungsvorgangs mit betriebsbereit gekoppelten Bauteilen: Strahlungsbauteil und Messküvette.

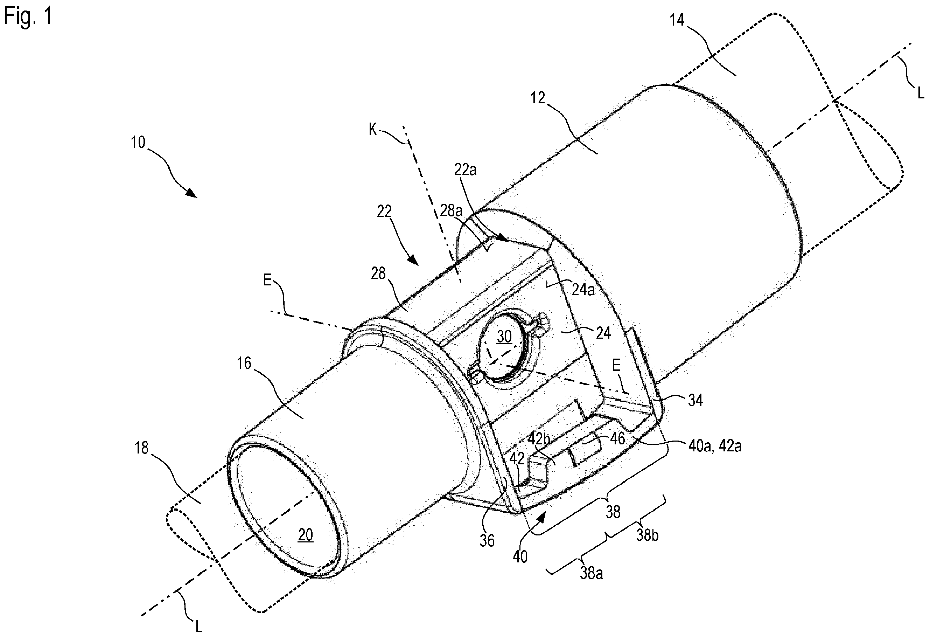

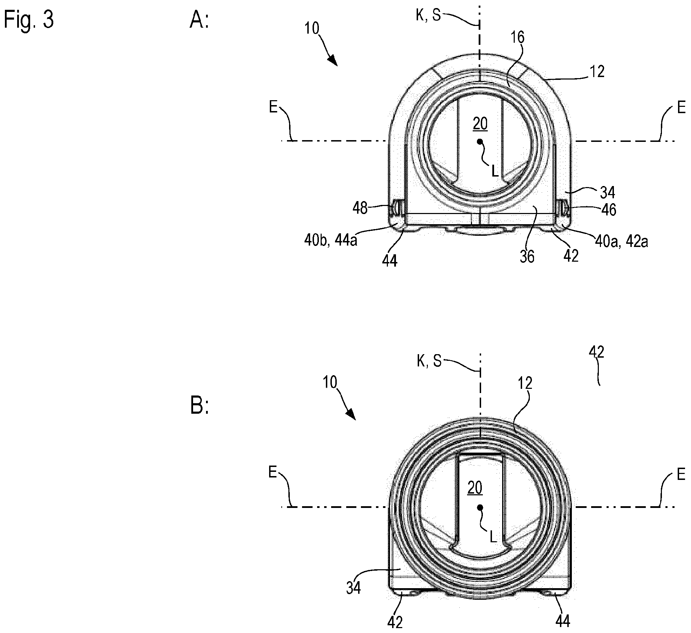

In den Figuren 1 bis 4B ist eine erfindungsgemäße Ausführungsform einer Messküvette der vorliegenden Anmeldung allgemein mit 10 bezeichnet. Sie umfasst an ihren beiden Längsenden eine erste Anschlussformation 12, welche zum Anschluss eines in Figur 1 lediglich strichliniert angedeuteten ersten Fluidleitungsschlauchs oder -rohrs 14 ausgebildet ist, sowie eine zweite Anschlussformation 16, welche zum Anschluss eines in Figur 1 lediglich strichliniert angedeuteten zweiten Fluidleitungsschlauchs oder -rohrs 18 ausgebildet ist. Die Anschlussformationen 12 und 16 können in an sich bekannter Weise ausgebildet sein, beispielsweise als Anschlussstutzen zur Steckverbindung, Bajonettverbindung oder Schraubverbindung. Die in Figur 1 dargestellte Messküvette 10 ist beispielsweise zur Anordnung in einer Atemgasleitung einer Beatmungsvorrichtung ausgebildet. Die Fluidleitungsschläuche 14 und 16 können daher Atemgasleitungsschläuche sein.

Im dargestellten Ausführungsbeispiel weist die erste Anschlussformation 12 einen größeren Außendurchmesser auf als die zweite Anschlussformation 16.

Die gesamte Messküvette 10 erstreckt sich in Längsrichtung längs einer geradlinigen Längsachse L, längs welcher die Messküvette 10 von Fluid durchströmbar ist. Die Messküvette 10 ist daher längs der Längsachse L durchsetzt von einem Kanal 20, von welchen in Figur 1 nur dessen Endbereich am Längsende der zweiten Anschlussformation 16 erkennbar ist.

Längs der Längsachse L zwischen den beiden Anschlussformationen 12 und 16 ist ein Kopplungs- und Erfassungsabschnitt 22 ausgebildet, welcher zwei zueinander und zur Längsachse L parallele Seitenwände 24 und 26 sowie eine die beiden Seitenwände 24 und 26 miteinander verbindende Basiswand 28 aufweist. Die Außenflächen 24a, 26a und 28a der jeweiligen Wände 24, 26 und 28 sind wenigstens abschnittsweise eben, wobei die Außenfläche 28a der Basiswand 28 orthogonal zu den Außenflächen 24a und 26a der an die Basiswand 28 anschließenden Seitenwände 24 und 26 orientiert ist. Auch die Basiswand 28 und ihre Außenfläche 28a sind bevorzugt eben ausgebildet und parallel zur Längsachse L orientiert.

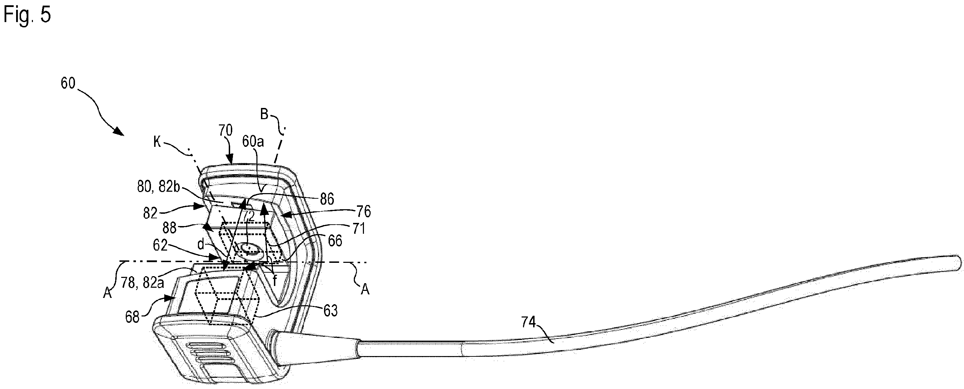

Aufgrund der Gestaltung der Seitenwände 24 und 26 als Wände mit zueinander parallelen ebenen Außenflächen 24a und 26a sowie weiter aufgrund der Ausgestaltung der Anschlussformationen 12 und 16 als konische oder zylindrische Formationen mit größeren Abmessungen orthogonal zur Längsachse L als der Kopplungs-und Erfassungsabschnitt 22 kann der Kopplungs- und Erfassungsabschnitt 22 nur in einer Richtung mit dem in Figur 5 gezeigten Strahlungsbauteil 60 gekoppelt werden, nämlich längs der in Figur 1 gezeigten, zur Längsachse L orthogonalen und zu den Außenflächen 24a und 26a parallelen Kopplungsachse K.

Es sei an dieser Stelle angemerkt, dass die Außenflächen 24a und 26a zur Bildung einer Einführschräge abweichend vom oben Gesagten mit zur Basiswand 28 hin abnehmenden Abstand voneinander ausgebildet sein können, um ein Einführen des Kopplungs- und Erfassungsabschnitts 22 in den Aufnahmeraum 62 des Strahlungsbauteils 60 zu erleichtern. In diesem Falle ist die Kopplungsachse K weiterhin orthogonal zur Längsachse L und bildet eine Winkelhalbierende des von den Außenflächen 24a und 26a eingeschlossenen Einführschrägenwinkels.

Die Seitenwände 24 und 26 weisen jeweils ein sie in Dickenrichtung durchsetzendes Fenster 30 bzw. 32 auf, welches für elektromagnetische Strahlung, im dargestellten Beispiel Infrarotstrahlung, vom Strahlungsbauteil 60 durchlässig ist. Der Kopplungs-und Erfassungsabschnitt 22 ist somit im betriebsbereit gekoppelten Zustand von Messküvette 10 und Strahlungsbauteil 60 von elektromagnetischer Strahlung durch die Fenster 30 und 32 hindurch längs einer sowohl zur Längsachse L als auch zur Kopplungsachse K orthogonalen Einstrahlachse E in an sich bekannter Weise durchstrahlbar.

Die erste Anschlussformation 12 endet zum Kopplungs- und Erfassungsabschnitt 22 hin in einem bezüglich der Kopplungsachse von der Basiswand 28 fernliegenden unteren Bereich in einem zur Längsachse L im Wesentlichen orthogonalen Wandabschnitt 34. Die zweite Anschlussformation 16 endet zum Kopplungs- und Erfassungsabschnitt 22 hin in einem zur Längsachse L im Wesentlichen orthogonalen Wandabschnitt 36. Der Kopplungs- und Erfassungsabschnitt 22 erstreckt sich nur zwischen diesen Wandabschnitten 34 und 36 zwischen seinen Längsenden 22a und 22b.

In dem Längserstreckungsabschnitt 38, in welchem sich der Kopplungs- und Erfassungsabschnitt 22 längs der Längsachse L erstreckt, befindet sich eine Orientierungsstruktur 40 der Messküvette 10, von welcher in Figur 1 nur eine Teilstruktur 40a zu erkennen ist.

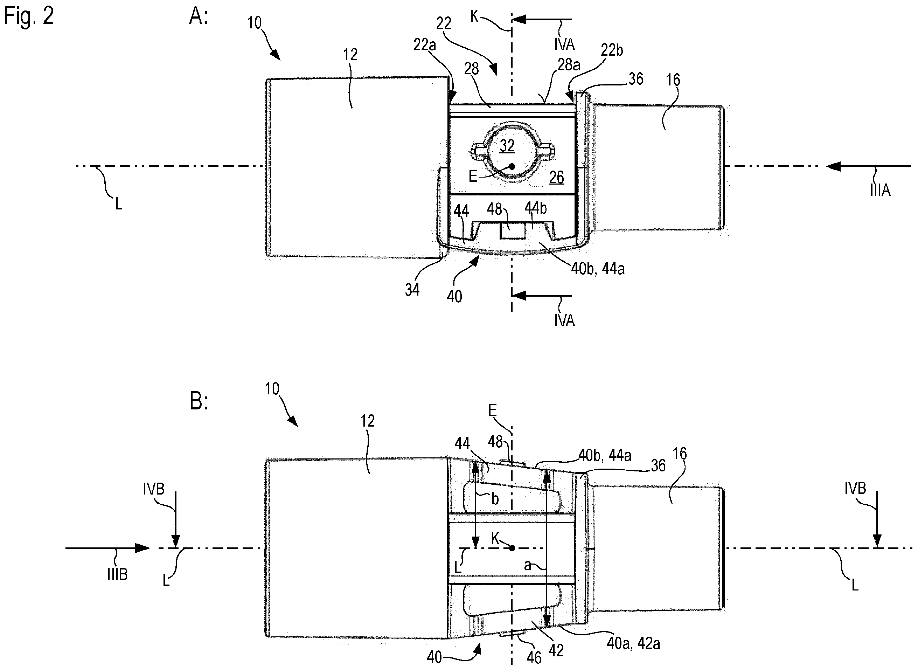

Die Teilstruktur 40a, welche ein Randabschnitt 42a eines Stegs 42 ist, weist in dem Koordinatensystem der Achsen L, K und E ihre größte Erstreckung längs der Längsachse L auf. Wie jedoch besonders gut in Figur 2B zu erkennen ist, ist die Teilstruktur 40a bezüglich der Längsachse L geneigt, und zwar um eine zur Kopplungsachse K parallele Neigeachse.

In Figur 2B ist die die Teilstruktur 40a zur Orientierungsstruktur 40 ergänzende weitere Teilstruktur 40b zu erkennen, welche bezüglich der Teilstruktur 40b spiegelsymmetrisch bezüglich einer die Längsachse L und die Kopplungsachse K enthaltenden Spiegelsymmetrieebene S ausgebildet ist. Auch die Teilstruktur 40a ist ein Randabschnitt 44a eines Stegs 44, welcher ebenfalls bezüglich der Spiegelsymmetrieebene S spiegelbildlich zum Steg 42 ausgebildet ist.

Ganz grundsätzlich ist vorzugsweise die gesamte Messküvette 10, nicht nur die im Ausführungsbeispiel dargestellte Orientierungsstruktur 40 bezüglich der Spiegelsymmetrieebene S spiegelsymmetrisch ausgebildet.

Die Orientierungsstruktur 40, gebildet durch ihre beschriebenen Teilstrukturen 40a und 40b weist somit eine zur Längsachse L orthogonale Abmessung a auf, welche sich längs der Längsachse L ändert, im dargestellten Beispiel über den gesamten Längserstreckungsabschnitt 38 hinweg und sogar beiderseits darüber hinaus. Es gibt also zwei längs der Längsachse L unmittelbar aufeinanderfolgende Abschnitte 38a und 38b, in welchen der zwischen den Teilstrukturen 40a und 40b bestehende zur Längsachse L orthogonale Abstand a unterschiedlich ist. Wegen der im Beispiel gezeigten kontinuierlichen Verjüngung der Orientierungsstruktur 40 längs der Längsachse L bestehen im dargestellten Beispiel tatsächlich unendlich viele solche unmittelbar aufeinanderfolgenden Abschnitte. Anstelle einer kontinuierlichen Verjüngung könnte sich der Abstand zwischen den Teilstrukturen 40a und 40b jedoch wenigstens abschnittsweise auch stufenweise ändern. Dann wäre die Anzahl an unmittelbar aufeinanderfolgenden Abschnitten mit unterschiedlicher Abmessung orthogonal zur Längsachse L endlich und entspräche bevorzugt der Anzahl der Stufen.

Wegen der oben beschriebenen spiegelbildlichen Ausbildung ändert sich auch längs der Längsachse L der Abstand b zwischen der den Kanal 20 zentral durchsetzend gedachten Längsachse L und jeder der Teilstrukturen 40a und 40b. Somit könnte aufgrund des sich längs der Längsachse L ändernden Abstands b auch jeder einzelne der Stege 42 oder 44 eine Orientierungsstruktur sein, obwohl sich die Abmessung des Stegs 42 und des Stegs 44 orthogonal zur Längsachse L längs der Längsachse L im dargestellten Beispiel nicht ändert. Der Abstand b ist bevorzugt sowohl zur Längsachse L als auch zur Kopplungsachse K orthogonal.

An beiden Stegen 42 und 44 ist je eine Zunge 42b bzw. 44b in Richtung der Kopplungsachse K zur selben Seite hin auskragend ausgebildet, auf welcher bezüglich der Stege 42 und 44 auch der Kanal 20 im Kopplungs- und Erfassungsabschnitt 22 vollständig gelegen ist. An diesen Zungen 42b und 44b ist eine längs der Einstrahlachse E abstehende Rastnase 46 bzw. 48 ausgebildet, welche zur überwindbaren Verrastung der Messküvette 10 am Strahlungsbauteil 60 im betriebsbereit gekoppelten Zustand ausgebildet sind.

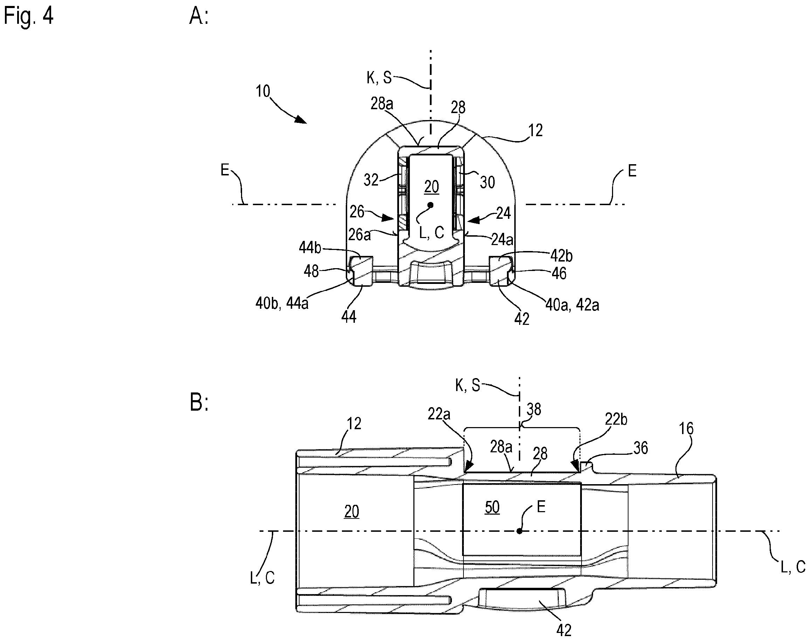

Die Figur 4B zeigt einen vom Kopplungs- und Erfassungsabschnitt 22 eingefassten Kanalbereich 50. Das ist jener Bereich des Kanals 22, welcher im Längserstreckungsbereich 38 des Kopplungs- und Erfassungsabschnitt 22 gelegen ist. Trotz der Änderungen in der Querschnittsgestalt und -fläche ist die Längsachse L auch eine den Kanal 20 zentral durchsetzende virtuelle Kanalbahn C.

Figur 5 zeigt das mit der Messküvette 10 zu koppelnde Strahlungsbauteil 60 in grobschematischer perspektivischer Unteransicht. Ein Aufnahmeraum 62 durchsetzt das Strahlungsbauteil 60 längs einer Aufnahmeachse A vollständig. Das Strahlungsbauteil 60 umgibt den Aufnahmeraum 62 von drei Seiten. Der Aufnahmeraum 62 ist derart ausgebildet, dass der Kopplungs- und Erfassungsabschnitt 22 der Messküvette 10 darin Platz hat, wobei zur Aufnahmeachse A und zueinander parallele Seitenwände 64 und 66 des Aufnahmeraums 62 bei betriebsbereiter Kopplung des Strahlungsbauteils 60 mit der Messküvette 10 mit den Seitenwänden 24 bzw. 26 des Kopplungs- und Erfassungsabschnitts 22 in Berührkontakt stehen.

Orthogonal zur Aufnahmeachse A in Tiefenrichtung des Aufnahmeraums 62 verläuft die Kopplungsachse K, welche für das Strahlungsbauteil 60 und die Messküvette 10 identisch ist. Orthogonal sowohl zur Aufnahmeachse A als auch zur Kopplungsachse K verläuft die Strahlungsachse B, längs welcher sowohl der Aufnahmeraum 62 als auch - im Falle der betriebsbereiten Kopplung des Strahlungsbauteils 60 mit der Messküvette 10 - der Kanalbereich 50 der Messküvette 10 mit elektromagnetischer Strahlung, vorzugsweise mit Infrarotstrahlung, durchstrahlbar ist.

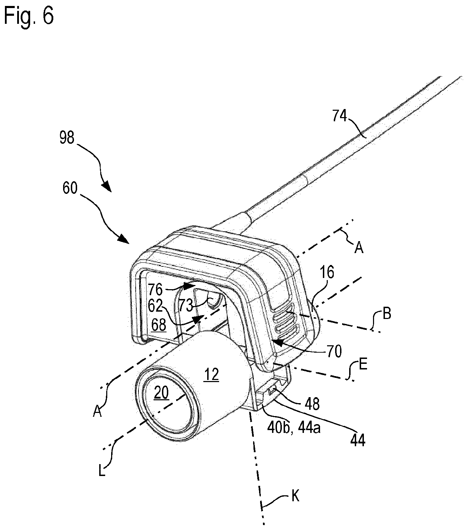

In dem größeren Kompartiment 68 auf einer Seite des Aufnahmeraums 62 ist eine nur in Figur 5 strichliniert angedeutete Strahlungsquelle 63 aufgenommen. In dem jenseits des Aufnahmeraums 62 dem größeren Kompartiment 68 gegenüberliegenden kleineren Kompartiment 70 befindet sich eine nur in Figur 5 strichliniert angedeutete Sensoreinrichtung 71, welche die von der Strahlungsquelle ausgestrahlte elektromagnetische Strahlung nach Durchgang durch den Aufnahmeraum 62 bzw. durch den Kanalbereich 50 erfasst. In Figur 5 ist ein Messfenster 72 erkennbar, durch welches hindurch elektromagnetische Strahlung zur Sensoreinrichtung im Kompartiment 70 gelangen kann. In Figur 6 ist ein Strahlungsfenster 73 erkennbar, durch welches die elektromagnetische Strahlung von der Strahlungsquelle ausgehend in den Aufnahmeraum 62 eintritt.

Durch ein Kabel 74 können Steuerbefehle zum Strahlungsbauteil 60 und Erfassungssignale von diesem zu einer übergeordneten Steuereinheit oder/und zu einer Auswerteeinheit übertragen werden.

Zwischen dem Aufnahmeraum 62 und der Außenfläche 60a des Strahlungsbauteils 60 ist eine Vertiefung 76 ausgebildet, welche an den beiden bezüglich der Aufnahmeachse A endseitigen Bereichen des Strahlungsbauteils 60 zur möglichst spielfreien Aufnahme des Wandabschnitts 36 auf der einen Seite sowie der ersten Anschlussformation 12 und des Wandabschnitts 34 ausgebildet ist.

Im Erstreckungsbereich des Aufnahmeraums 62 ist die Vertiefung 76 beiderseits des Aufnahmeraums 62 durch je eine Flanke 78 und 80 begrenzt. Jede dieser Flanken 78 und 80 bildet eine Teilstruktur 82a bzw. 82b einer Ausrichtungsstruktur 82, welche aufgrund ihrer Ausgestaltung geeignet und bestimmt ist, eine betriebsbereite Kopplung der Messküvette 10 mit ihrer Orientierungsstruktur 40 mit dem Strahlungsbauteil 60 nur in genau einer gewünschten Relativorientierung zuzulassen.

Jede der Teilstrukturen 82a und 82b verläuft hauptsächlich, d. h. mit ihrer größten Erstreckungskomponente im kartesischen Koordinatensystem der Achsen K, A und B, längs der Aufnahmeachse A, ist jedoch bezüglich der Aufnahmeachse A geneigt, vorzugsweise wenigstens um eine zur Kopplungsachse K parallele Neigeachse. So, wie die Randabschnitte 42a und 44a, die die Orientierungsstruktur 40 der Messküvette 10 bilden, im Wesentlichen ebene Randabschnitte sind, sind auch die Flanken 78 und 80 im wesentlichen ebene Flanken. Die Flanke 78 liegt im betriebsbereit gekoppelten Zustand von Messküvette 10 und Strahlungsbauteil 60 flächig am Randabschnitt 42a an. Die Flanke 80 am Randabschnitt 44a. Jede Flanke 78 und 80 weist je eine Rastausnehmung als Verrastungsmittel auf, wobei in Figur 5 nur die Rastausnehmung 86 der Flanke 80 sichtbar ist. Im betriebsbereit gekoppelten Zustand der Sensorbaugruppe 98 aus Messküvette 10 und Strahlungsbauteil 60 greift die Rastnase 46 der Messküvette 10 in die Rastausnehmung der Flanke 78 und greift die Rastnase 48 der Messküvette 10 in die Rastausnehmung 86 der Flanke 80 formschlüssig zur Herstellung eines überwindbaren Rasteingriffs ein.

Die Ausrichtungsstruktur 82 verjüngt sich kontinuierlich längs der Aufnahmeachse A, d. h. der Abstand d zwischen den einander über den Aufnahmeraum 62 hinweg gegenüberliegenden Flanken 78 und 80 wird längs der Aufnahmeachse A kontinuierlich kleiner bzw. größer, je nachdem in welcher Richtung man die Aufnahmeachse A durchläuft. Ebenso ändert sich der Abstand f einer jeden Flanke 78 und 80 zur Aufnahmeachse A längs der Aufnahmeachse A kontinuierlich. Deshalb könnte auch jede der Flanken 78 und 80 für sich alleine genommen eine Ausrichtungsstruktur sein. Wegen der im Beispiel gewählten spiegelsymmetrischen Ausbildung der Messküvette 10 bzw. ihres Kopplungs- und Erfassungsabschnitts 22 sind die beiden Abstände f der Flanken 78 und 80 zur Aufnahmeachse A an jeder Stelle längs der Aufnahmeachse A betragsmäßig gleich groß. Dies muss jedoch nicht so sein.

Der Aufnahmeraum 62 ist orthogonal zur Aufnahmeachse A nur in einer Richtung längs der Kopplungsachse K offen. Dort befindet sich die Einführöffnung 88, durch welche hindurch der Kopplungs- und Erfassungsabschnitt 22 der Messküvette 10 in den Aufnahmeraum 62 einführbar ist. Durch die Einführöffnung 88 ist der Kopplungs-und Erfassungsabschnitt 22 auch wieder aus dem Aufnahmeraum 62 entnehmbar.

In Figur 6 ist ein Zustand der Sensorbaugruppe 98 gezeigt, bei welchem der Kopplungs- und Erfassungsabschnitt 22 der Messküvette 10 in einem Kopplungsvorgang durch die Einführöffnung 88 in den Aufnahmeraum 62 längs der Kopplungsachse K eingeführt wird, jedoch noch nicht betriebsbereit eingeführt und gekoppelt ist. Die Kopplungsachse K der Messküvette 10 und des Strahlungsbauteils 60 sind kollinear. Die Aufnahmeachse A und die Längsachse L sind zueinander parallel, jedoch mit Abstand voneinander angeordnet. Ebenso sind die Strahlungsachse B und die Einstrahlachse E parallel, jedoch mit Abstand voneinander angeordnet.

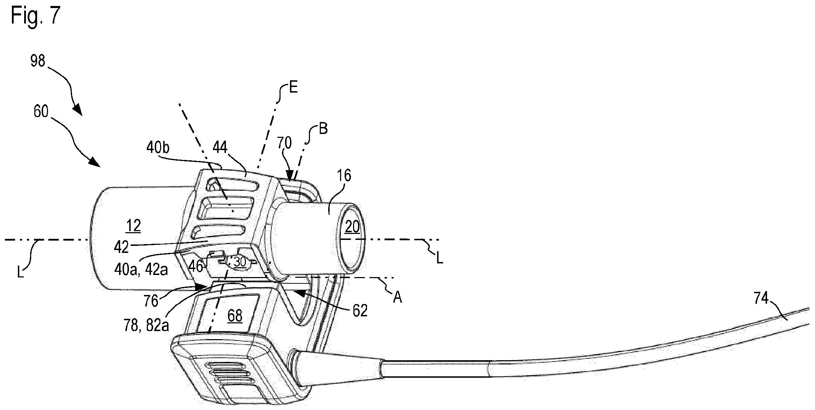

In Figur 7 ist der Zustand der Sensorbaugruppe 98 von Figur 6 in einer grobschematischen Unteransicht dargestellt. In Figur 7 ist am besten zu erkennen, wie die zueinander komplementär ausgebildeten Strukturen: Orientierungsstruktur 40 und Ausrichtungsstruktur 82, eine betriebsbereite Kopplung der Messküvette 10 und des Strahlungsbauteils 60 nur in genau einer gewünschten Relativorientierung zulassen.

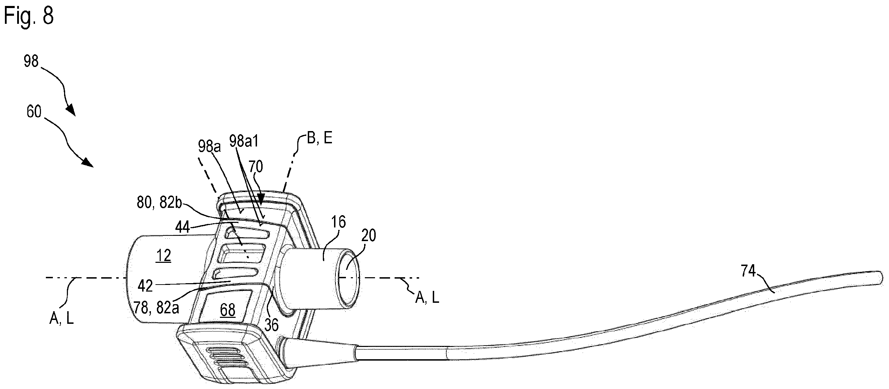

In Figur 8 ist bei identischer Blickrichtung wie in Figur 7 der betriebsbereit gekoppelte Zustand von Messküvette 10 und Strahlungsbauteil 60 dargestellt. Zu erkennen ist, wie die Außenseiten der Stege 42 und 44 der Messküvette 10, die auch die Orientierungsstruktur 40 ausbilden, im betriebsbereit gekoppelten Zustand mit dem Strahlungsbauteil 60 über die Trennfuge zwischen der Orientierungsstruktur 40 und der Ausrichtungsstruktur 82 hinweg eine bündigen Bereich 98a1 der Außenfläche 98a der Sensorbaugruppe 98 bilden.

## Patentansprüche

1. Messküvette (10) zur Erfassung wenigstens eines Fluidbestandteils eines die Messküvette (10) durchströmenden Fluids unter Beteiligung von elektromagnetischer Strahlung, die Messküvette (10) umfassend:
- einen die Messküvette (10) längs einer Kanalbahn (C) durchsetzenden Kanal (20), wobei die Kanalbahn (C) als den Kanal (20) zentral durchsetzend gedacht ist,
- eine erste Anschlussformation (12) zum Anschluss einer ersten Fluidförderleitung (14),
- eine zweite Anschlussformation (16) zum Anschluss einer zweiten Fluidförderleitung (18),
- zwischen der ersten und der zweiten Anschlussformation (12, 16) einen Kopplungs- und Erfassungsabschnitt (22), welcher
• sich zwischen seinem der ersten Anschlussformation (12) näheren ersten Längsende (22a) und seinem der zweiten Anschlussformation (16) näheren zweiten Längsende (22b) längs einer geradlinigen Längsachse (L) erstreckt,
• wenigstens zur Einstrahlung von elektromagnetischer Strahlung in einen vom Kopplungs- und Erfassungsabschnitt (22) umgebenen Kanalbereich (50) der Messküvette (10) ausgebildet ist und
• zur lösbaren mechanischen Kopplung mit einem Strahlungsbauteil (60), umfassend eine Strahlungsquelle (63) oder/und einen Strahlungssensor (71) der elektromagnetischen Strahlung, ausgebildet ist,
wobei die Messküvette (10) eine körperliche Orientierungsstruktur (40) aufweist, die eine lösbare mechanische Kopplung der Messküvette (10) mit dem Strahlungsbauteil (60) in einer vorbestimmten gewünschten Relativorientierung gestattet und in einer unerwünschten Relativorientierung verhindert,
wobei die körperliche Orientierungsstruktur (40) zwischen dem ersten und dem zweiten Längsende (22a, 22b) des Kopplungs- und Erfassungsabschnitts (22) mit einem sich längs der Längsachse (L) ändernden Abstand (b) zu der Längsachse (L) oder/und mit einer sich längs der Längsachse (L) ändernden, zur Längsachse (L) orthogonalen Abmessung (a) angeordnet und ausgebildet ist,
**dadurch gekennzeichnet, dass** sich die Orientierungsstruktur (40) längs der Längsachse (L) über mehr als ein Drittel der Abmessung des Kopplungs- und Erfassungsabschnitts (22) erstreckt.

2. Messküvette (10) nach Anspruch 1,
**dadurch gekennzeichnet, dass** die Orientierungsstruktur (40) wenigstens zwei Abschnitte (38a, 38b) mit unterschiedlichem Abstand (b) zur Längsachse oder/und mit unterschiedlicher zur Längsachse (L) orthogonaler Abmessung (a) aufweist, wobei die Abschnitte (38a, 38b) längs der Längsachse (L) lückenlos aufeinander folgen.

3. Messküvette (10) nach einem der vorherigen Ansprüche,
**dadurch gekennzeichnet, dass** der Kopplungs- und Erfassungsabschnitt (22) zwei längs der Längsachse (L) verlaufende, orthogonal zur Längsachse (L) mit Abstand voneinander angeordnete Wände (24, 26) aufweist, welche den Kanalbereich (50) der Messküvette (10) begrenzen, wobei die Orientierungsstruktur (40) einen längs der Längsachse (L) verlaufenden Randabschnitt (42a, 44a) aufweist, welcher bezüglich der Längsachse (L) geneigt ist.

4. Messküvette (10) nach Anspruch 3,
**dadurch gekennzeichnet, dass** die Orientierungsstruktur (40) zwei in einem gemeinsamen Längsbereich (38) längs der Längsachse (L) verlaufende Rand abschnitte (42a, 44a) aufweist, auf jeder Seite des Kanalbereichs (50) je einen, wobei jeder der zwei Randabschnitte (42a, 44a) bezüglich der Längsachse (L) geneigt ist, wobei vorzugsweise sich der orthogonal zur Längsachse (L) zu messende Abstand (a) der beiden Randabschnitte (42a, 44a) voneinander längs der Längsachse (L) ändert, besonders bevorzugt kontinuierlich ändert.

5. Messküvette (10) nach einem der vorherigen Ansprüche,
**dadurch gekennzeichnet, dass** die Orientierungsstruktur (40) Rastmittel (46, 48) trägt, welche zur Verrastung mit Verrastungsmitteln (86) am Strahlungsbauteil (60) ausgebildet sind.

6. Messküvette (10) nach Anspruch 5, unter Rückbeziehung auf einen der Ansprüche 3 oder 4,
**dadurch gekennzeichnet, dass** die Rastmittel (46, 48) im Bereich des wenigstens einen Randabschnitts (42a, 44a) vorgesehen sind, vorzugsweise bezüglich des wenigstens einen Randabschnitts (42a, 44a) zu derselben Seite hin vom wenigstens einen Randabschnitt (42a, 44a) abstehend, auf welcher sich der unter Beteiligung der beiden Wände (24, 26) begrenzte Kanalbereich (50) der Messküvette (10) befindet.

7. Strahlungsbauteil (60), ausgebildet zur Kopplung mit einer Messküvette (10) nach einem der vorhergehenden Ansprüche, wobei das Strahlungsbauteil (60)
einen Aufnahmeraum (62) von drei Seiten umgibt, in welchem der Kopplungs-und Erfassungsabschnitt (22) der Messküvette (10) aufnehmbar ist, wobei wenigstens ein den Aufnahmeraum (62) begrenzender Abschnitt (72, 73) des Strahlungsbauteils (60) für elektromagnetische Strahlung durchlässig ausgebildet ist, so dass der Aufnahmeraum (62) für von einer Strahlungsquelle (63) im Strahlungsbauteil (60) ausgehende elektromagnetische Strahlung erreichbar ist, wobei der Aufnahmeraum (62) das Strahlungsbauteil (60) längs einer zentral durch den Aufnahmeraum (62) verlaufend gedachten geradlinigen Aufnahmeachse (A) durchsetzt, wobei das Strahlungsbauteil (60) eine körperliche Ausrichtungsstruktur (82) aufweist, welche eine lösbare mechanische Kopplung des Strahlungsbauteils (60) mit der Messküvette (10) in einer vorbestimmten gewünschten Relativausrichtung gestattet und in einer unerwünschten Relativausrichtung verhindert,
wobei die körperliche Ausrichtungsstruktur (82) am Strahlungsbauteil (60) im Erstreckungsbereich des Aufnahmeraums (62) mit einem sich längs der Aufnahmeachse (A) ändernden Abstand (f) zu der Aufnahmeachse (A) oder/und mit einer sich längs der Aufnahmeachse (A) ändernden zur Aufnahmeachse (A) orthogonalen Abmessung (d) angeordnet und ausgebildet ist,
**dadurch gekennzeichnet, dass** sich die Ausrichtungsstruktur (82) längs der Aufnahmeachse (A) über mehr als ein Drittel der Abmessung des Aufnahmeraums (62) erstreckt.

8. Strahlungsbauteil (60) nach Anspruch 7,
**dadurch gekennzeichnet, dass** die Ausrichtungsstruktur (82) wenigstens zwei Abschnitte mit unterschiedlichem Abstand (f) zur Aufnahmeachse (A) oder/und mit unterschiedlicher zur Aufnahmeachse (A) orthogonaler Abmessung (d) aufweist, wobei die Abschnitte längs der Aufnahmeachse (A) lückenlos aufeinander folgen.

9. Strahlungsbauteil (60) nach einem der Ansprüche 7 oder 8,
**dadurch gekennzeichnet, dass** an einem Gehäuse des Strahlungsbauteils (60) wenigstens im Bereich einer im Wesentlichen parallel zur Aufnahmeachse (A) verlaufenden, das Strahlungsbauteil (60) vollständig durchsetzenden Einführöffnung (88) eine an die Einführöffnung (88) angrenzende Vertiefung (76) ausgebildet ist, wobei die Ausrichtungsstruktur (82) an oder in der Vertiefung (76) ausgebildet ist.

10. Strahlungsbauteil (60) nach Anspruch 9,
**dadurch gekennzeichnet, dass** die Ausrichtungsstruktur (82) eine die Vertiefung (76) begrenzende Flanke (78, 80) umfasst oder ist.

11. Strahlungsbauteil (60) nach Anspruch 10,
**dadurch gekennzeichnet, dass** die Ausrichtungsstruktur (82) beiderseits der Einführöffnung (88) je eine die Vertiefung (76) begrenzende Flanke (78, 80) umfasst oder ist, wobei bevorzugt der orthogonal zur Aufnahmeachse (A) zu messende Abstand (d) der Flanken sich längs der Aufnahmeachse (A) ändert, besonders bevorzugt kontinuierlich ändert.

12. Strahlungsbauteil (60) nach einem der Ansprüche 7 bis 11,
**dadurch gekennzeichnet, dass** das Strahlungsbauteil (60) Verrastungsmittel (86) zur Verrastung der Messküvette (10) im betriebsbereiten Zustand aufweist.

13. Strahlungsbauteil (60) nach Anspruch 12, unter Rückbeziehung auf einen der Ansprüche 10 oder 11,
**dadurch gekennzeichnet, dass** die Verrastungsmittel (86) in der wenigstens einen Flanke (78, 80) angeordnet oder ausgebildet sind.

14. Sensorbaugruppe (98), umfassend eine Messküvette (10) nach einem der Ansprüche 1 bis 6 und ein Strahlungsbauteil (60) nach einem der Ansprüche 7 bis 13, wobei im betriebsbereiten Zustand, in welchem die Messküvette (10) und das Strahlungsbauteil (60) lösbar miteinander gekoppelt sind, die Längsachse (L) und die Aufnahmeachse (A) in einem gemeinsamen Raumbereich parallel oder kollinear zueinander sind, wobei die Orientierungsstruktur (40) und die Ausrichtungsstruktur (82) zumindest abschnittsweise, vorzugsweise vollständig, in Formschluss- oder Anlageeingriff miteinander stehen.

15. Sensorbaugruppe (98) nach Anspruch 14,
**dadurch gekennzeichnet, dass** im betriebsbereiten Zustand ein die Orientierungsstruktur (40) aufweisender küvettenseitiger Bauteilabschnitt (42, 44) und ein die Ausrichtungsstruktur (82) aufweisender strahlungsbauteilseitiger Bauteilabschnitt an der Anlagestelle von Orientierungsstruktur (40) und Ausrichtungsstruktur (82) einen bündigen Außenflächenbereich (98a1) der Sensorbaugruppe (98) bilden.

## Claims

1. Measuring cuvette (10) for detecting at least one fluid component of a fluid flowing through the measuring cuvette (10) with the involvement of electromagnetic radiation, the measuring cuvette (10) comprising:
- a duct (20) penetrating through the measuring cuvette (10) along a duct path (C), where the duct path (C) is conceived as penetrating through the duct (20) centrally,
- a first connector formation (12) for connecting a first fluid-conveying line (14),
- a second connector formation (16) for connecting a second fluid-conveying line (18),
- a coupling and detection section (22) between the first and the second connector formation (12, 16), which
• Extends along a straight longitudinal axis (L) between its first longitudinal end (22a) which is nearer to the first connector formation (12) and its second longitudinal end (22b) which is nearer to the second connector formation (16),
• Is configured at least for irradiation of electromagnetic radiation into a duct region (50) of the measuring cuvette (10) surrounded by the coupling and detection section (22), and
• Is configured for detachable mechanical coupling with a radiation component (60), comprising a radiation source (63) and/or a radiation sensor (71) of the electromagnetic radiation,
where the measuring cuvette (10) exhibits a physical orientation structure (40) which allows detachable mechanical coupling of the measuring cuvette (10) with the radiation component (60) in a predetermined desirable relative orientation and prevents it in an undesirable relative orientation,
wherein the physical orientation structure (40) is arranged and configured between the first and the second longitudinal end (22a, 22b) of the coupling and detection section (22) at a distance (b) to the longitudinal axis (L) which varies along the longitudinal axis (L) and/or with a dimension (a) orthogonally to the longitudinal axis (L) which varies along the longitudinal axis (L),
**characterized in that** the orientation structure (40) extends along the longitudinal axis (L) over more than one third of the dimension of the coupling and detection section (22).

2. Measuring cuvette (10) according to Claim 1,
**characterized in that** the orientation structure (40) exhibits at least two sections (38a, 38b) with different distances (b) to the longitudinal axis and/or with different dimensions (a) orthogonally to the longitudinal axis (L), where the sections (38a, 38b) follow one another without gaps along the longitudinal axis (L).

3. Measuring cuvette (10) according to one of the preceding Claims,
**Characterized in that** the coupling and detection section (22) exhibits two walls (24, 26) extending along the longitudinal axis (L), arranged orthogonally to the longitudinal axis (L) at a distance from one another, which bound the duct region (50) of the measuring cuvette (10), where the orientation structure (40) exhibits an edge section (42a, 44a) extending along the longitudinal axis (L) which is tilted relative to the longitudinal axis (L).

4. Measuring cuvette (10) according to Claim 3,
**Characterized in that** the orientation structure (40) exhibits two edge sections (42a, 44a) in a common longitudinal region (38) extending along the longitudinal axis (L), one each on each side of the duct region (50), where each of the two edge sections (42a, 44a) is tilted relative to the longitudinal axis (L), where preferably the distance (a) of the two edge sections (42a, 44a) from one another measured orthogonally to the longitudinal axis (L) varies along the longitudinal axis (L), especially preferably varies continuously.

5. Measuring cuvette (10) according to one of the preceding Claims,
**Characterized in that** the orientation structure (40) carries latching elements (46, 48) which are configured for latching with locking elements (86) on the radiation component (60).

6. Measuring cuvette (10) according to Claim 5, by reference to one of the Claims 3 or 4,
**Characterized in that** the latching elements (46, 48) are provided in the region of the at least one edge section (42a, 44a), preferably projecting relative to the at least one edge section (42a, 44a) from the at least one edge section (42a, 44a) towards the same side on which the duct region (50) of the measuring cuvette (10) which is bounded with the involvement of the two walls (24, 26) is situated.

7. Radiation component (60), configured for coupling with a measuring cuvette (10) according to one of the preceding Claims, where the radiation component (60) surrounds on three sides an accommodating space (62) in which the coupling and detection section (22) of the measuring cuvette (10) can be accommodated, where at least one section (72, 73) of the radiation component (60) which bounds the accommodating space (62) is configured as transparent to electromagnetic radiation such that the accommodating space (62) is reachable for electromagnetic radiation emitted from a radiation source (63) in the radiation component (60), where the accommodating space (62) penetrates through the radiation component (60) along a straight accommodation axis (A) conceived as extending centrally through the accommodating space (62), where the radiation component (60) exhibits a physical aligning structure (82) which allows detachable mechanical coupling of the radiation component (60) with the measuring cuvette (10) in a predetermined desirable relative alignment and prevents it in an undesirable relative alignment,
wherein the physical aligning structure (82) is arranged and configured at the radiation component (60) in the extension region of the accommodating space (62) at a distance (f) to the accommodation axis (A) which varies along the accommodation axis (A) and/or with a dimension (d) orthogonally to the accommodation axis (A) which varies along the accommodation axis (A),
**characterized in that** the aligning structure (82) extends along the accommodation axis (A) over more than a third of the dimension of the accommodating space (62).

8. Radiation component (60) according to Claim 7,
**characterized in that** the aligning structure (82) exhibits at least two sections at different distances (f) to the accommodation axis (A) and/or with different dimensions (d) orthogonally to the accommodation axis (A), where the sections follow one another without gaps along the accommodation axis (A).

9. Radiation component (60) according to one of the Claims 7 or 8,
**characterized in that** at a housing of the radiation component (60) at least in the region of an insertion aperture (88) extending essentially in parallel to the accommodation axis (A), penetrating completely through the radiation component (60) there is configured a recess (76) bordering the insertion aperture (88), where the aligning structure (82) is configured at or in the recess (76).

10. Radiation component (60) according to Claim 9,
**characterized in that** the aligning structure (82) comprises or is a flank (78, 80) bounding the recess (76).

11. Radiation component (60) according to Claim 10,
**characterized in that** the aligning structure (82) comprises or is one flank (78, 80) each bordering the recess (76) on both sides of the insertion aperture (88), where preferably the spacing (d) of the flanks measured orthogonally to the accommodation axis (A) varies along the accommodation axis (A), especially preferably varies continuously.

12. Radiation component (60) according to one of the Claims 7 to 11,
**characterized in that** the radiation component (60) exhibits locking elements (86) for locking the measuring cuvette (10) in the operational state.

13. Radiation component (60) according to Claim 12, by reference to one of the Claims 10 or 11,
**characterized in that** the locking elements (86) are arranged or configured in the at least one flank (78, 80).

14. Sensor assembly (98), comprising a measuring cuvette (10) according to one of the Claims 1 to 6 and a radiation component (60) according to one of the Claims 7 to 13, where in the operational state, in which the measuring cuvette (10) and the radiation component (60) are coupled detachably with one another, the longitudinal axis (L) and the accommodation axis (A) are parallel or collinear with one another in a common spatial region, where the orientation structure (40) and the aligning structure (82) are at least section-wise, preferably completely, in positive-locking or abutment engagement with one another.

15. Sensor assembly (98) according to Claim 14,
**Characterized in that** in the operational state a cuvette-side component section (42, 44) exhibiting the orientation structure (40) and a radiation component-side component section exhibiting the aligning structure (82) form at the abutment point of the orientation structure (40) and the aligning structure (82) a flush outer surface region (98a1) of the sensor assembly (98).

## Revendications

1. Cuvette de mesure (10) pour la détection d'au moins un composant de fluide d'un fluide traversant la cuvette de mesure (10) avec participation d'un rayonnement électromagnétique, la cuvette de mesure (10) comprenant :
- un canal (20) traversant la cuvette de mesure (10) le long d'une voie de canal (C), dans lequel la voie de canal (C) est conçue comme traversant le canal (20) de manière centrale,
- une première formation de raccordement (12) pour le raccordement d'une première conduite de transport de fluide (14),
- une deuxième formation de raccordement (16) pour le raccordement d'une deuxième conduite de transport de fluide (18),
- entre la première et la deuxième formation de raccordement (12, 16), une section de couplage et de détection (22) qui
• s'étend le long d'un axe longitudinal rectiligne (L) entre sa première extrémité longitudinale (22a) plus proche de la première formation de raccordement (12) et sa deuxième extrémité longitudinale (22b) plus proche de la deuxième formation de raccordement (16),
• est conçue au moins pour l'irradiation d'un rayonnement électromagnétique dans une zone de canal (50) de la cuvette de mesure (10) entourée par la section de couplage et de détection (22) et
• est conçue pour être couplée mécaniquement de manière amovible à un composant de rayonnement (60), comprenant une source de rayonnement (63) ou/et un capteur de rayonnement (71) du rayonnement électromagnétique,
dans lequel la cuvette de mesure (10) présente une structure d'orientation corporelle (40) qui permet un couplage mécanique amovible de la cuvette de mesure (10) avec le composant de rayonnement (60) dans une orientation relative souhaitée prédéterminée et qui l'empêche dans une orientation relative non souhaitée,
dans lequel la structure d'orientation physique (40) est disposée et réalisée entre la première et la deuxième extrémité longitudinale (22a, 22b) de la section de couplage et de détection (22) à une distance (b) de l'axe longitudinal (L) qui varie le long de l'axe longitudinal (L) ou/et avec une dimension (a) orthogonale à l'axe longitudinal (L) qui varie le long de l'axe longitudinal (L),
**caractérisé en ce que** la structure d'orientation (40) s'étend le long de l'axe longitudinal (L) sur plus d'un tiers de la dimension de la section de couplage et de détection (22).

2. Cuvette de mesure (10) selon la revendication 1,
**caractérisée en ce que** la structure d'orientation (40) présente au moins deux sections (38a, 38b) avec une distance (b) différente par rapport à l'axe longitudinal ou/et avec une dimension (a) différente orthogonale à l'axe longitudinal (L), dans lequel les sections (38a, 38b) se succèdent sans espace le long de l'axe longitudinal (L).

3. Cuvette de mesure (10) selon l'une des revendications précédentes,
**caractérisée en ce que** la section de couplage et de détection (22) présente deux parois (24, 26) s'étendant le long de l'axe longitudinal (L), disposées orthogonalement à l'axe longitudinal (L) et à distance l'une de l'autre, qui délimitent la zone de canal (50) de la cuvette de mesure (10), dans lequel la structure d'orientation (40) présente une section de bord (42a, 44a) s'étendant le long de l'axe longitudinal (L), qui est inclinée par rapport à l'axe longitudinal (L).

4. Cuvette de mesure (10) selon la revendication 3,
**caractérisée en ce que** la structure d'orientation (40) présente deux sections de bord (42a, 44a) s'étendant dans une zone longitudinale commune (38) le long de l'axe longitudinal (L), une de chaque côté de la zone de canal (50), dans lequel chacune des deux sections de bord (42a, 44a) est inclinée par rapport à l'axe longitudinal (L), dans lequel la distance (a) des deux sections de bord (42a, 44a) à mesurer orthogonalement à l'axe longitudinal (L) varie de préférence le long de l'axe longitudinal (L), et varie de préférence en continu.

5. Cuvette de mesure (10) selon l'une des revendications précédentes,
**caractérisée en ce que** la structure d'orientation (40) porte des moyens d'encliquetage (46, 48) qui sont conçus pour s'encliqueter avec des moyens d'encliquetage (86) sur le composant de rayonnement (60).

6. Cuvette de mesure (10) selon la revendication 5, en référence à l'une des revendications 3 ou 4,
**caractérisée en ce que** les moyens d'encliquetage (46, 48) sont prévus dans la zone de ladite au moins une section de bord (42a, 44a), de préférence en saillie par rapport à ladite au moins une section de bord (42a, 44a) du même côté de ladite au moins une section de bord (42a, 44a) sur laquelle se trouve la zone de canal (50) de la cuvette de mesure (10) délimitée avec la participation des deux parois (24, 26).

7. Composant de rayonnement (60) conçu pour être couplé à une cuvette de mesure (10) selon l'une des revendications précédentes, dans lequel le composant de rayonnement (60) entoure de trois côtés un espace de réception (62) dans lequel la section de couplage et de détection (22) de la cuvette de mesure (10) peut être reçue, dans lequel au moins une section (72, 73) du composant de rayonnement (60) délimitant l'espace de réception (62) est réalisée de manière à être perméable au rayonnement électromagnétique, de sorte que l'espace de réception (62) peut être atteint par le rayonnement électromagnétique provenant d'une source de rayonnement (63) dans le composant de rayonnement (60), dans lequel l'espace de réception (62) traverse le composant de rayonnement (60) le long d'un axe de réception (A) rectiligne imaginaire s'étendant au centre de l'espace de réception (62), dans lequel le composant de rayonnement (60) présente une structure d'orientation physique (82) qui permet un couplage mécanique amovible du composant de rayonnement (60) avec la cuvette de mesure (10) dans une orientation relative souhaitée prédéterminée et l'empêche dans une orientation relative non souhaitée, dans lequel la structure d'orientation physique (82) est disposée et réalisée sur le composant de rayonnement (60) dans la zone d'extension de l'espace de réception (62) à une distance (f) de l'axe de réception (A) qui varie le long de l'axe de réception (A) ou/et avec une dimension (d) orthogonale à l'axe de réception (A) qui varie le long de l'axe de réception (A),
**caractérisé en ce que** la structure d'alignement (82) s'étend le long de l'axe de réception (A) sur plus d'un tiers de la dimension de l'espace de réception (62).

8. Composant de rayonnement (60) selon la revendication 7,
**caractérisé en ce que** la structure d'alignement (82) présente au moins deux sections avec une distance (f) différente par rapport à l'axe de réception (A) ou/et avec une dimension (d) orthogonale différente par rapport à l'axe de réception (A), dans lequel les sections se succèdent sans espace le long de l'axe de réception (A).

9. Composant de rayonnement (60) selon l'une des revendications 7 ou 8,
**caractérisé en ce qu'**un creux (76) adjacent à l'ouverture d'introduction (88) est formé sur un boîtier du composant de rayonnement (60) au moins dans la zone d'une ouverture d'introduction (88) s'étendant sensiblement parallèlement à l'axe de réception (A) et traversant complètement le composant de rayonnement (60), dans lequel la structure d'orientation (82) est formée sur ou dans le creux (76).

10. Composant de rayonnement (60) selon la revendication 9,
**caractérisé en ce que** la structure d'alignement (82) comprend ou est un flanc (78, 80) délimitant le creux (76).

11. Composant de rayonnement (60) selon la revendication 10,
**caractérisé en ce que** la structure d'orientation (82) comprend ou est un flanc (78, 80) délimitant le creux (76) de chaque côté de l'ouverture d'introduction (88), dans lequel de préférence la distance (d) des flancs à mesurer orthogonalement à l'axe de réception (A) varie le long de l'axe de réception (A), de préférence varie en continu.

12. Composant de rayonnement (60) selon l'une des revendications 7 à 11,
**caractérisé en ce que** le composant de rayonnement (60) présente des moyens d'encliquetage (86) pour encliqueter la cuvette de mesure (10) à l'état prêt à l'emploi.

13. Composant de rayonnement (60) selon la revendication 12, en référence à l'une des revendications 10 ou 11,
**caractérisé en ce que** les moyens d'encliquetage (86) sont disposés ou formés dans ledit au moins un flanc (78, 80).

14. Ensemble de capteur (98) comprenant une cuvette de mesure (10) selon l'une des revendications 1 à 6 et un composant de rayonnement (60) selon l'une des revendications 7 à 13, dans lequel, à l'état prêt à l'emploi où la cuvette de mesure (10) et le composant de rayonnement (60) sont couplés l'un à l'autre de manière amovible, l'axe longitudinal (L) et l'axe de réception (A) sont parallèles ou colinéaires l'un par rapport à l'autre dans une zone spatiale commune, dans lequel la structure d'orientation (40) et la structure d'alignement (82) sont au moins en partie, de préférence entièrement, en engagement par complémentarité de forme ou d'appui l'une avec l'autre.

15. Ensemble de capteur (98) selon la revendication 14,
**caractérisé en ce que**, à l'état prêt à l'emploi, une section de composant (42, 44) côté cuvette présentant la structure d'orientation (40) et une section de composant côté composant de rayonnement présentant la structure d'orientation (82) forment, au point d'appui de la structure d'orientation (40) et de la structure d'alignement (82), une zone de surface extérieure (98a1) affleurante de l'ensemble de capteur (98).
